(19) Europäisches Patentamt
European Patent Office
Office européen des brevets

(11) Publication number: 0 200 436
A2

# (12) EUROPEAN PATENT APPLICATION

(21) Application number: 86302933.6

(22) Date of filing: 18.04.86

(51) Int. Cl.4: C 07 D 471/14
A 61 K 31/44
//(C07D471/14, 221:00, 221:00, 209:00)

(30) Priority: 19.04.85 HU 151885

(43) Date of publication of application:
05.11.86 Bulletin 86/45

(84) Designated Contracting States:
AT BE CH DE FR GB IT LI LU NL SE

(71) Applicant: RICHTER GEDEON VEGYESZETI GYAR R.T.
Gyömröi ut 19-21
H-1103 Budapest X(HU)

(72) Inventor: Szántay, Csaba
Zsombolyai u. 8
H-113 Budapest(HU)

(72) Inventor: Incze, Mária
Cserje u. 18
H-1025 Budapest(HU)

(72) Inventor: Szombathelye, Zsolt
Munkácsy M. u. 12
H-1063 Budapest(HU)

(72) Inventor: Sóti, Ferenc
Szász Károly u. 4
H-1027 Budapest(HU)

(72) Inventor: Balogh, Zsuzsanna
Galgóczy Köz 7/b
H-1125 Budapest(HU)

(72) Inventor: Kárpati, Egon
Mihályfi E. u. 7b
H-1022 Budapest(HU)

(72) Inventor: Kiss, Béla
OTP 1tp A/1
H-2220 Vevsés(HU)

(72) Inventor: Csomor, Katsalin
Nyár u. 99
H-1045 Budapest(HU)

(72) Inventor: Laszlovsky, István
Bartók Béla ut 16
H-1111 Budapest(HU)

(72) Inventor: Lapis, Erzsébet
Abaliget u. 86
H-1172 Budapest(HU)

(72) Inventor: Szporny, László
Szabolcska M. u. 7
H-1114 Budapest(HU)

(72) Inventor: Forgács, Lilla
Galamb u. 7
H-1052 Budapest(HU)

(72) Inventor: Kuthi, Csaba
Bürök u. 24
H-1124 Budapest(HU)

(74) Representative: Pett, Christopher Phineas et al,
Frank B. Dehn & Co. European Patent Attorneys Imperial House 15-19 Kingsway
London WC2B 6UZ(GB)

(54) 12b-Substituted 1-hydroxymethyl-octahydroindolo]2,3-a[quinolizine derivatives.

(57) The invention relates to new racemic and optically active 12b-substituted 1-hydroxymethyl-octahydroindolo[2,3-a]quinolizine derivatives of formula (I)

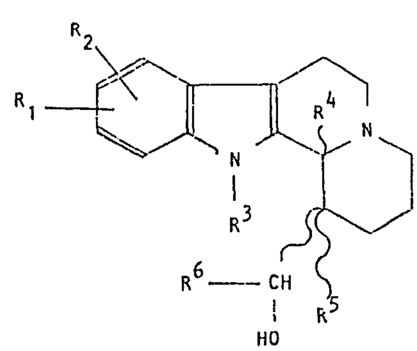

(I)

./...

EP 0 200 436 A2

wherein

$R^1$ and $R^2$ are the same or different and represent hydrogen, halogen, nitro, amino, hydroxyl, alkyl having from 1 to 6 carbon atoms or alkoxy having from 1 to 6 carbon atoms;

$R^3$ and $R^5$ are the same or different and represent hydrogen, alkyl having from 1 to 6 carbon atoms or aralkyl having from 1 to 6 carbon atoms in the alkyl moiety;

$R^4$ is alkyl having from 1 to 6 carbon atoms, aryl or aralkyl having from 1 to 6 carbon atoms in the alkyl moiety;

$R^6$ is hydrogen or alkyl having from 1 to 6 carbon atoms, aryl or aralkyl having from 1 to 6 carbon atoms in the alkyl moiety, and the isomers and acid addition salts thereof.

The compounds of the invention are valuable as intermediates in the preparation of pharmaceutically active compounds and are pharmaceutically active themselves.

IU 50 450

# 12b-Substituted 1-hydroxymethyl-octahydroindolo-[2,3-a]-quinolizine derivatives

The invention relates to new racemic and optically active 12b-Substituted 1-hydroxymethyl-octahydroindolo-[2,3-a]-quinolizine derivatives. Such compounds display useful cardiovascular activity and are also useful in the preparation of further active compounds.

Compounds of the invention and their preparation are new. The literature only discloses 12b-substituted 1,2,3,4,6,7,12, 12b-octahydroindolo-[2,3-a]-quinolizine compounds which do not contain a hydroxymethyl or substituted hydroxymethyl group on the 1- carbon atom which is characteristic of the compounds of the present invention.

According to one aspect the present invention provides 12b-substituted 1-hydroxymethyl-octahydroindolo-[2,3-a]-quinolizine derivatives of formula (I)

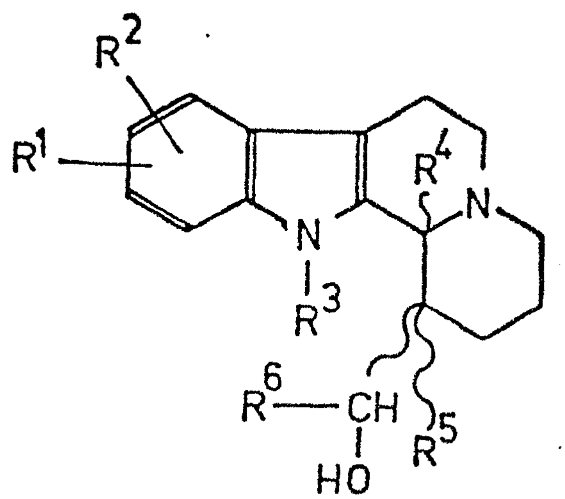

(I)

wherein

$R^1$ and $R^2$ are the same or different and represent hydrogen, halogen, nitro, amino, hydroxyl, alkyl having from 1 to 6 carbon atoms

or alkoxy having from 1 to 6 carbon atoms;

$R^3$ and $R^5$ are the same or different and represent hydrogen, alkyl having from 1 to 6 carbon atoms or aralkyl having from 1 to 6 carbon atoms in the alkyl moiety;

$R^4$ is alkyl having from 1 to 6 carbon atoms, aryl or aralkyl having from 1 to 6 carbon atoms in the alkyl moeity;

$R^6$ is hydrogen or alkyl having from 1 to 6 carbon atoms, aryl or aralkyl having from 1 to 6 carbon atoms in the alkyl moiety,

and optically-active isomers, racemic mixtures and acid additional salts thereof.

Preferred compounds of formula (I) are selected from the group of:

1-hydroxymethyl-12b-methyl-1,2,3,4,6,7,12,12b-octahydroindolo[2,3-a]quinolizine and its ethanesulfonate;

1-hydroxymethyl-12,12b-dimethyl-1,2,3,4,6,7,12,12b-octahydroindolo[2,3-a]quinolizine;

1-hydroxymethyl-1,12,12b-trimethyl-1,2,3,4,6,7,12,12b-octahydroindolo[2,3-a]quinolizine;

1-(1-hydroxymethyl)-12b-methyl-1,2,3,4,6,7,12,12b-octahydroindolo[2,3-a]quinolizine;

9-bromo-, 10-bromo- and 8,9-dibromo-1-hydroxymethyl-12b-methyl-1,2,3,4,6,7,12,12b-octahydroindolo[2,3-a]quinolizine;

8-nitro- and 10-nitro-1-hydroxy-methyl-12b-methyl-1,2,3,4,6,7,12,12b-octahydroindolo[2,3-a]quinolizine; and

10-amino-1-(hydroxymethyl)-12b-methyl-1,2,3,4,6,7,12,12b-octahydroindolo[2,3-a]quinolizine,

and racemic mixtures and optical antipodes thereof, cis- and trans-isomers thereof, and mixtures of such antipodes and isomers.

The compounds of formula (I) are valuable intermediates in the preparation of other pharmaceutically active compounds, and are pharmaceutically active themselves. In particular they show cardiovascular, especially peripheral vasodilating activity.

According to another aspect, the invention provides a pharmaceutical composition comprising as active ingredient at least one optically active 12b-substituted 1-hydroxymethyl-octahydroindolo-[2,3-a]-quinolizine derivative of formula (I) or racemic mixtures thereof, as hereinbefore defined or an isomer or a physiologically acceptable acid addition salt thereof, in association with a pharmaceutically acceptable carrier, diluent and/or excipient.

According to a still further aspect of the present invention there is provided a process for the preparation of optically active 12b-substituted 1-hydroxymethyl-octahydroindolo-[2,3-a]-quinolizine derivatives as hereinbefore defined comprising

reducing an optically active 1,12b-disubstituted -octahydroindolo-[2,3-a]quinolizine derivative of formula (II)

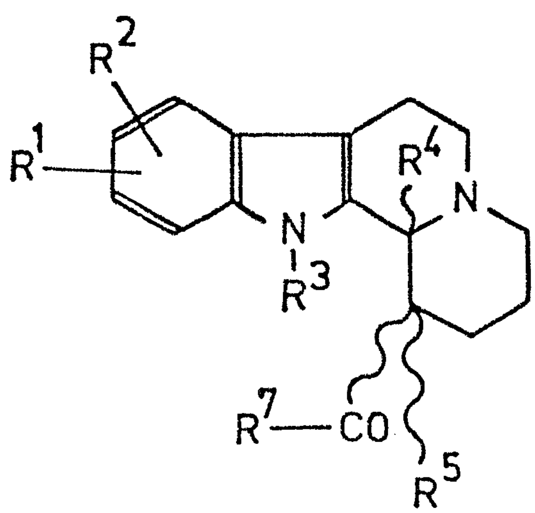

(II)

wherein

$R^1$, $R^2$ are as hereinbefore defined and

$R^7$ is alkoxy having from 1 to 6 carbon atoms, alkyl having from 1 to 6 carbon atoms, aryl or

aralkyl having from 1 to 6 carbon atoms in the alkyl moiety,

or racmic mixture thereof; followed, if desired by

subjecting a 12b-substituted 1-hydroxymethyl-octahydroindolo-[2,3-a]-quinolizine derivative of formula (I) obtained, in which $R^1$ and/or $R^2$ is halogen or a nitro group and/or $R^3$ represent a benzyl group, and $R^4$, $R^5$ and $R^6$ are as defined hereinbefore to further reduction,

and/or, if desired treating a 12b-substituted 1-hydroxymethyl-octahydroindolo-[2,3-a]quinolizine derivative of formula (I) obtained, in which $R^1$ $R^2$, $R^3$, $R^4$, $R^5$ and $R^6$ are as hereinbefore defined with an acid,

or treating an acid addition salt of a 12b-substituted 1-hydroxymethyl-octahydroindolo-[2,3-a]quinolizine derivative of formula (I) obtained, wherein $R^1$ $R^2$, $R^3$, $R^4$, $R^5$ and $R^6$ are as hereinbefore defined with a base,

and/or, if desired, separating an isomeric mixture obtained into the respective isomers,

and/or if desired resolving a racemic 12b-substituted 1-hydroxymethyl-octahydroindolo[2,3-a]quinolizine derivative of formula (I) obtained.

In the above formulae, where $R^1$ and $R^2$ are halogen they may represent any halogen atom, such as fluorine, chlorine, bromine or iodine;

when $R^1$, $R^2$, $R^3$, $R^4$, $R^5$, $R^6$ and $R^7$ are an alkyl group having from 1 to 6 carbon atoms they may represent a straight-chained or branched alkyl group having from 1 to 6 carbon atoms, e.g. methyl, ethyl, _n_-propyl, isopropyl, _n_-butyl, sec.-butyl, isobutyl, tert.-butyl, _n_-pentyl, isopentyl, _n_-hexyl, isohexyl, etc.;

when $R^1$, $R^2$ and $R^7$ are an alkoxy having from 1 to 6 carbon atoms, they are the oxy-derivatives of any of the above-identified alkyl groups;

in the definition of $R^3$, $R^4$, $R^5$, $R^6$ and $R^7$ the term "aralkyl having from 1 to 6 carbon atoms in the alkyl moiety" is used to define a combination of any of the above alkyl groups with a mono- or multi-cyclic, isolated or condensed aromatic group, e.g. phenyl, diphenyl, naphthyl, etc.;

when $R^4$, $R^6$ and $R^7$ are an "aryl" group they may be any of the aryl groups defined in connection with the aralkyl groups.

According to the invention, compounds of formula (I) are prepared starting from 12b-substituted 1,2,3,4,6,7,12, 12b-octahydroindolo-[2,3-a]-quinolizine derivatives, in which a carbonyl group is attached to the 1-carbon atom. This carbonyl group, among others, may be part of an ester or acyl group. These compounds may be encompassed by formula (II). The preparation of 12b-substituted 1-carbonyl-1,2,3,4, 6,7,12, 12b-octahydroindolo-[2,3-a]quinolizine derivatives of formula (II) is disclosed in our co-pending Hungarian Patent Application No. 1517/85. Using this procedure the starting compounds of formula (II) are prepared from an appropriately substituted tryptamine derivative with a corresponding ketone, by condnsation and subsequent cyclization.

According to the invention the reduction of the compounds of formula (II) is carried out in an inert organic solvent. The reduction may be performed using different methods, depending on the substituents $R^1$, $R^2$, $R^3$ and $R^7$. If in the starting compound of formula (II), $R^3$ is benzyl, the compound is sensitive to catalytic hydrogenation.

If in the starting compound of formula (II) $R^7$ represents an alkoxy group, the eventual $R^1$ and $R^2$ substituents are not sensitive to reduction, i.e. are other than halogen or nitro and $R^3$, $R^4$ and $R^5$ are as hereinbefore defined the reduction is preferably carried out with the strong reducing

agent lithium aluminium hydride. According to a preferred embodiment of this process the indolo-[2,3-a]quinolizine derivative of formula (II), preferably as a solution in tetrahydrofuran, is added to a suspension of lithium aluminium hydride in a conventional solvent such as a linear or cyclic ether, e.g. ether, tetrahydrofuran, dioxan; a tertiary base, e.g. pyridine; preferably tetrahydrofuran at a temperature between room temperature and the boiling point of the solvent employed, more preferably at room temperature. The product of formula (I) is isolated in a known manner, by treating the reaction mixture with an aqueous alkaline solution, filtering off the aluminium hydroxide formed, drying the solvent phase and evaporating off the solvent. If desired, the product obtained may be further purified by column chromatography or recrystallization.

If $R^7$ represents an alkoxy group, $R^1$ and/or $R^2$ is a substituent sensitive to reduction with lithium aluminium hydride, e.g. halogen or nitro and $R^3$, $R^4$ and $R^5$ are as hereinbefore defined, the reducing agent should be selected so that it should reduce the 1-alkoxycarbonyl group without a simultaneous reduction of $R^1$ and/or $R^2$. In this case

the reduction is preferably performed with sodium borohydride in the presence of aluminium chloride /‾ this type of reduction is described in: H.C. Brown, B.C. S.Rao: J.Am.Chem.Soc., 78, 2582 (1956)7. According to a preferred embodiment of this process the indolo/2,3-a7quinolizine derivative to be reduced is added to a solution or suspension of sodium borohydride in a linear or cyclic ether, e.g. diethyleneglycol dimethyl ether, tetrahydrofurane, ether or in a mixture thereof, preferably in diethyleneglycol dimethyl ether, followed by the addition of aluminium chloride, preferably as a solution in diethyleneglycol dimethyl ether, at a temperature between room temperature and 75 °C, preferably at room temperature. The reduction is complete in about 1 to 3 hours. Thereafter, the reaction mixture is poured into a mixture of concentrated hydrochloric acid and ice and the reduced product of the formula (I) is isolated after making the reaction mixture alkaline, preferably with sodium carbonate. The isolation may be carried out for example by filtration. If desired, the crude compound of the formula (I) may be further purified by chromatography or recrystallization.

If in the compounds of the formula (II) $R^7$ is an alkyl, aryl or aralkyl group, i.e. if the compound is a ketone, the reduction may be carried out also under considerably milder conditions, i.e. with a milder reducing agent. In this case the

reduction may also be carried out with sodium borohydride by Meerwein-Ponndorf reduction [for the method see: T.Bersin: Angew. Chem., 53, 266 (1940) and A.L.Wilds: Org. Reactions 2, 178 (1948)], or if in the starting compound of the formula (II) $R^1$ and/or $R^2$ and/or $R^3$ are not sensitive to catalytic hydrogenation, catalytic hydrogenation may also be employed.

If compounds of the formula (I), in which $R^1$ and/or $R^2$ stands for an amino group are to be prepared, and $R^6$ represents hydrogen, and in the starting compounds of the formula (II) $R^1$ and/or $R^2$ is a nitro group and $R^7$ is alkoxy, the reduction is carried out with a complex metal hydride, preferably with a mixture of lithium aluminium hydride and aluminium chloride. If, however, in the starting compounds of the formula (II) $R^1$ and/or $R^2$ is a nitro group, and $R^7$ is alkyl, aryl or aralkyl and compounds of the formula (I), in which $R^1$ and/or $R^2$ is amino, and $R^6$ is identical with $R^7$ are to be prepared, the reduction is performed with catalytically activated hydrogen, preferably using Raney nickel, palladium-on-charcoal or platinum as a catalyst.

Compounds of the formula (I), in which $R^3$ is hydrogen may be prepared from compounds of the formula (II), in which $R^3$ is benzyl and $R^1$ and $R^2$ are not sensitive to catalytic hydrogenation, by reduction with catalytically activated hydrogen, as described

above. The $R^3$ benzyl substituent may be removed also by reduction in liquid ammonia with sodium metal.

When the compounds of the formula (II) are reduced with sodium borohydride, the indolo[2,3-a]-quinolizine derivative is preferably dissolved in a lower alcohol having from 1 to 6 carbon atoms, preferably methanol, and solid sodium borohydride is added to the solution in portions, at a temperature between 0 °C and the boiling point of the solvent employed, preferably at room temperature. When the reduction is complete, the excess of sodium borohydride is decomposed with acetone, the reaction mixture is evaporated, the residue is treated with water and a water-immiscible solvent, preferably dichloromethane or ether, and the product is extracted with the selected water-immiscible organic solvent. The product of the formula (I) obtained by evaporation of the latter extract can be further purified by recrystallization or column chromatography.

If the reduction of a compound of the formula (II) is carried out by Meerwein-Ponndorf reduction, the indolo[2,3-a]quinolizine derivative is preferably dissolved in an aliphatic alcohol having from 1 to 6 carbon atoms or in a mixture thereof with an aromatic hydrocarbon, e.g. ethanol, isopropanol, a mixture of isopropanol and benzene, preferably isopropanol, aluminium alkoxide, preferably aluminium isopropoxide catalyst is added to the solution, and the reaction

mixture is slightly boiled, while isopropanol and the acetone formed are allowed to distill off slowly. When the reaction is complete, i.e. when no acetone can be detected in the distillate, the reaction mixture is completely evaporated, the aluminium alkoxide is treated with aqueous sodium hydroxide solution and the product is extracted with a water-immiscible solvent, preferably a halogenated aliphatic hydrocarbon, an ether-type solvent, preferably dichloromethane, chloroform, ether, preferably chloroform. From the extract the product can be isolated in a conventional manner, e.g. by evaporation, and if desired, can be further purified e.g. by crystallization or column chromatography.

If the compounds of formula (II) are reduced catalytically, the starting compound of the formula (II) is preferably dissolved in an inert organic solvent, preferably a lower aliphatic alcohol having from 1 to 6 carbon atoms, such as ethanol and the solution is admixed or shaken with hydrogen gas in the presence of a Raney nickel, palladium-on-charcoal or platinum catalyst. After termination of the reduction, the catalyst is removed, preferably by filtration and the product is isolated by evaporation of the solution. If desired, the crude product may be further purified by chromatography or recrystallization.

If in the starting indolo/2,3-a7quinolizine derivatives $R^7$ represents an alkyl, aryl or aralkyl

group, the indolo/2,3-a/quinolizine derivatives of formula (I) are formed as a result of the reduction in the form of epimeric mixtures related to the asymmetric carbon atoms also originally present in the molecule. One of the epimers may be predominant. If desired, the epimeric mixture may be separated into its components, by crystallization in the form of a base or an acid addition salt, or by chromatography of the base.

If desired, the racemic indolo/2,3-a/quinolizine compounds of formula (I) obtained by the process according to the invention may be separated into their optical isomers. During the resolution generally an acid addition salt of an indolo/2,3-a/quinolizine derivative of formula (I) with an optically active acid is generally separated into a mixture of diastereomers by crystallization. An optically active acid for example tartaric acid, diacyl tartaric acid or a semidialkyl amide of the latter compound, e.g. dibenzoyl-(+)-tartaric acid semidimethyl amide, malic acid, camphenic acid or the substituted derivatives of camphenic acid etc., can be used. Typical solvents include lower aliphatic alcohols, preferably having from 1 to 6 carbon atoms. If both antipodes of a compound of the formula (I) are to be prepared by resolution, from the salt resulting from the evaporation of the mother liquor obtained after separation of one of the two isomers, the base enriched in the other antipode is decanted, and

the other antipode is isolated by further resolution with a resolving agent, preferably with the antipode of the resolving agent used in the first step. The optically active indolo[2,3-a]quinolizine derivatives of formula (I) be liberated from the diastereomeric salt pair obtained, if desired. For this purpose the salt is dissolved or suspended in a mixture of water and a water-immiscible organic solvent, such as an optionally halogenated aliphatic or aromatic hydrocarbon, a linear or cyclic ether, e.g. dichloromethane, chloroform, ether, toluene, preferably dichloromethane, the solution or suspension is made alkaline with an inorganic base, such as ammonia, sodium carbonate, potassium carbonate, preferably sodium carbonate, and the optically active base is extracted with the water-immiscible solvent employed. The pure optically active indolo[2,3-a]-quinolizine base of formula (I) is isolated from the solution e.g. by evaporation and if desired, by subsequent recrystallization.

The indolo[2,3-a]quinolizine derivatives of the formula (I) may, if desired, be converted into their acid addition salts, preferably physiologically acceptable acid addition salts by reaction with an inorganic or organic acid. Such acids include, amongst others, mineral acids such as, for example hydrohalic acids (e.g. hydrochloric or hydrobromic acids), sulfuric acid and phosphoric acid; organic carboxylic

acids such as for example formic acid, acetic acid, propionic acid, glycolic acid, maleic acid, fumaric acid, succinic acid, tartaric acid, ascorbic acid, citric acid, cinnamic acid, benzoic acid, malic acid, lactic acid, asparaginic acid, glutamic acid; methanesulfonic acid, ethanesulfonic acid, p-toluene-sulfonic acid, etc.

Salt formation can be carried out, for example, in an inert organic solvent such as a lower, preferably $C_{1-6}$ aliphatic alcohol, e.g. methyl, ethyl, n-propyl or isopropyl alcohol; in ethers such as ethyl ether, diisopropyl ether; cyclic ethers, e.g. dioxane, tetrahydrofurane or in other inert solvents such as ethyl acetate, acetone or in a mixture of one or more of these solvents, so that the compound of formula (I) is dissolved in the solvent or solvent mixture, and a calculated amount of the selected acid or a solution thereof formed with the same solvent is added to the first solution, preferably with cooling and stirring. Alternatively, to the solution of the compound of formula (I) in any of the above solvents or in a mixture thereof the selected acid or a solution thereof formed with the same solvent is added until it becomes slightly acidic (pH 5 to 6). Thereafter the acid addition salt separates out and may be removed from the reaction mixture e.g. by filtration. If desired, the isolated product may be further purified by recrystallization from any of the above solvents or from a suitable mixture thereof.

The vasodilating activity of the compounds of the formula (I) was tested on anaesthesized dogs. Electromagnetic rheometers (Hellige) were placed on the femoral and internal carotid arteries of the animals, and the blood flows measured in ml./min. The arterial mean pressure was measured using a Statham pressure sensor attached to a polyethylene cannula introduced into the artery. The pulse number per minute was determined from the pulsatoric component of the blood pressure using a frequency meter. All the measured parameters were continuously registered on a multichannel polygraph.

The activity of each compound was tested on more animals. The individual data obtained were averaged. In the following tables the number of animals (n), the mean values of the measured parameters and the percentage changes are indicated.

For intravenous administration the starting basic values and the maximum change were recorded. For intraduodenal administration (by means of a cannula introduced into the duodenum) the starting value and the observed change at further, given times ( every 5, 10, 30 minutes) were evaluated.

The test results are shown in Tables 1 and 2.

Table 1

The pharmacological activities of the compound prepared according to Example 2, 5 mg./kg. i.d. dose, administered 0. minute (n=7)

Blood flow in the femoral artery

| minutes | 0 | 5 | 10 | 20 | 30 | 60 | 90 | 120 |
|---|---|---|---|---|---|---|---|---|
| $ml.min^{-1}$ | 33 | 43 | 54 | 57 | 54 | 46 | 41 | 36 |
| % | - | +27 | +59 | +71 | +65 | +39 | +26 | +15 |

Blood flow in the internal carotid artery

| minutes | 0 | 5 | 10 | 20 | 30 | 60 | 90 | 120 |
|---|---|---|---|---|---|---|---|---|
| $ml.min^{-1}$ | 35 | 35 | 36 | 35.5 | 34.4 | 33.5 | 32.5 | 31.6 |
| % | - | 0 | +2.8 | +1.4 | +1.7 | -4.2 | -7.1 | -9.7 |

Arterial mean pressure

| minutes | 0 | 5 | 10 | 20 | 30 | 60 | 90 | 120 |
|---|---|---|---|---|---|---|---|---|
| kPa | 20.1 | 20.5 | 21.1 | 20.8 | 20.5 | 20.1 | 20.4 | 20.2 |
| % | - | +2 | +4.5 | +3 | +2 | 0 | +1.3 | +0.7 |

Pulse number

| minutes | 0 | 5 | 10 | 20 | 30 | 60 | 90 | 120 |
|---|---|---|---|---|---|---|---|---|
| $min^{-1}$ | 215 | 215 | 200 | 190 | 190 | 195 | 200 | 200 |
| % | - | 0 | -7 | -12 | -12 | -9 | -7 | -7 |

Table 2

Change of the blood flow in the femoral artery under the effect of 1 mg./kg. i.v. doses (n=5)

| Example 8 | | | Example 2 | | |
| --- | --- | --- | --- | --- | --- |
| basic | max. change | duration of act. | basic | max. change | duration of act. |
| | $ml.min^{-1}$ | min. | | $ml.min^{-1}$ | min. |
| 62 | 80 | 44 | 54 | 90 | 30 |
| % - | +28.7 | - | - | +66 | - |

| pentoxyfylline | | |
| --- | --- | --- |
| basic | max. change | duration of act. |
| | $ml.min.^{-1}$ | min. |
| 32.4 | 33.4 | 1.8 |
| % - | +3.1 | - |

As shown in Table 1, the compound prepared according to Example 2, administered in an i.d. dose of 5 mg./kg. substantially increases the femoral (extremital) blood flow (71 %) and has a lasting effect. Apart from a slight reduction in the pulse number it has practically no other cardiovascular effect. Accordingly, this compound shows a specific vasodilating activity.

In Table 2 the test results obtained by investigating the compounds according to Examples 8 and 2 and pentoxifylline, a compound widely used in the therapy as peripheral vasodilator (Trental$^R$) are set forth. The compounds were administered intravenously (i.v.) in 1 mg./kg. doses.

It can be seen that for i.v. administration both the compound according to Example 2 and the compound according to Example 8 have a vasodilating activity and this activity is substantially superior to that of pentoxifylline. The compound according to Example 2 proved to be particularly potent, it showed a substantial and long-lasting peripheral vasodilating activity for i.v. and i.d. administration. as well.

The compounds of the formula (I) can be advantageously used therapeutically in the treatment of diseases accompanied with vasoconstriction. In the case of parenteral administration their expected dose is 0.1 to 1.0 mg./kg. of body weight, while in case of oral administration 1 to 10 mg./kg. of body weight.

The new compounds of formula (I) and their physiologically acceptable acid addition salts may be formulated for therapeutic purposes. The invention therefore relates also to pharmaceutical compositions comprising at least one compound of formula (I) or a physiologically acceptable acid addition salt thereof, in association with pharmaceutical carriers and/or excipients. Carriers conventional

for this purpose and suitable for parenteral or enteral administration as well as other additives may be used. Solid or liquid compounds, for example water, gelatine, lactose, talc, vegetable oils such as peanut oil, olive oil, etc. can be used ascameirs The compounds can be formulated as conventional pharmaceutical formulations, for example in a solid (globular and angular pills, dragées, capsules, e.g. hard gelatine capsules) or liquid (e.g. oily or aqueous solutions, suspensions, emulsions, syrups, soft gelatine capsules, injectable oily or aqueous solutions or suspensions, etc.) form. The quantity of solid carrier may be varied within wide ranges, but preferably is between 25 mg. and 1 g. The compositions optionally also contain conventional pharmaceutical additives, such as preserving agents, stabilizers, wetting agents, emulsifying agents, salts for adjusting the osmotic pressure, buffers, flavouring and aroma-producing substances, etc.

The compositions according to the invention optionally contain the compounds of formula (I) in association with other known active ingredients. The unit doses are selected according to the route of administration. The pharmaceutical compositions are prepared by conventional techniques including sieving, mixing, granulation, pressing or dissolution of the ingredients. The formulations obtained may then be subjected to additional conventional treatments such as sterilization.

The invention is elucidated in detail using aid of the following non-limiting Examples.

Example 1

(±)-1β- Hydroxymethyl -12bβ-methyl-1,2,3,4,6,7,12,12b-octahydroindolo[2,3-a]quinolizine

To a suspension of 5.7 g. (0.15 mole) of lithium aluminium hydride in 150 ml. of dry tetrahydrofurane a solution of 8.55 g. (0.0274 mole) of (±)-ethyl 12bβ-methyl-1,2,3,4,6,7,12,12b-octahydroindolo[2,3-a]quinolizine-1β-carboxylate in 50 ml. of dry tetrahydrofurane is added dropwise, under argon atmosphere, at room temperature in 20 minutes, and the mixture is stirred at room temperature for one hour. Under ice cooling and stirring 5.7 ml. of water, 5.7 ml. of a 15 % aqueous sodium hydroxide solution and subsequently 17.1 ml. of water are added carefully, dropwise. The precipitate is filtered off, washed with three 20-ml. portions of tetrahydrofurane, the combined tetrahydrofurane solution is dried over solid anhydrous magnesium sulfate and evaporated.

Yield: 6.28 g. (85 %)

Melting point: 183 $^{o}$ to 185 $^{o}$C

$R_f$: 0.26 (Polygram SIL G/$UV_{254}$, ethyl acetate containing 10 % of triethylamine, u.v. light)

Example 2

(±)-1β- Hydroxymethyl -12bβ-methyl-1,2,3,4,6,7,12,12b-octahydroindolo/2,3-a/quinolizine ethanesulfonate

To a suspension of 4.59 g. (0.017 mole) of the base prepared according to Example 1 in 17 ml. of isopropyl alcohol a solution of 1.925 g. (0.0175 mole) of ethanesulfonic acid in 17 ml. of isopropyl alcohol is added under stirring, and when the dissolution is complete the mixture is diluted with 50 ml. of acetone. The product is obtained in a crystalline form upon rubbing.

Yield: 5.99 g. (93 %)

Melting point: 184 ° to 187 °C

Analysis for $C_{19}H_{28}N_2O_4S$ (380.49):

calculated: C 59.97 %, H 7.42 %, N 7.36 %, S 8.43 %;

found: C 59.82 %, H 7.21 %, N 7,39 %, S 8.39 %.

Example 3

(+)-1β- Hydroxymethyl -12bβ-methyl-1,2,3,4,6,7,12,12b-octahydroindolo/2,3-a/quinolizine

The title compound is prepared by the reduction of 8.02 g. (0.0257 mole) of (-)-12bβ-methyl-1,2,3,4,6,7,12,12b-octahydroindolo/2,3-a/quinolizine-1β-carboxylic acid ethyl ester with lithium aluminium hydride, following the procedure and using the reactants in the quantities described in Example 1.

Yield: 6.74 g. (97 %) of an oily product crystallizing upon standing

Melting point: 187 ° to 188 °C

$[\alpha]_D^{25}$= +17.4° (c=2.0; ethanol)

$R_f$: 0.26 (Polygram SIL G/$UV_{254}$, ethyl acetate containing 10 % of triethyl amine, u.v. light)

Example 4

(+)-1β- Hydroxymethyl -12bβ-methyl-1,2,3,4,6,7,12,12b-octahydroindolo[2,3-a]quinolizine ethanesulfonate

To a suspension of 6.48 g. (0.024 mole) of the base according to Example 3 in 10 ml. of isopropyl alcohol a solution of 2.64 g. (0.024 mole) of ethanesulfonic acid in 5 ml. of isopropyl alcohol is added, the mixture is boiled to complete dissolution and is subsequently diluted in portions with 200 ml. of acetone. The title compound is obtained as a crystalline product.

Yield: 7.79 g. (85 %)

Melting point: 238° to 242°C

$[\alpha]_D^{22}$= +15.4° (c=2.0; ethanol)

Analysis for $C_{19}H_{28}N_2O_4S$ (380.49):

| | | | | | | | | |
|---|---|---|---|---|---|---|---|---|
| calculated: | C | 59.97 %, | H | 7.42 %, | N | 7.36 %, | S | 8.43 %; |
| found: | C | 60.04 %, | H | 7.43 %, | N | 7.35 %, | S | 8.25 %. |

Example 5

(-)-1α- Hydroxymethyl -12bα-methyl-1,2,3,4,6,7,12,12b-octahydroindolo[2,3-a]quinolizine

The title compound is prepared by the reduction of 7.10 g. (0.0227 mole) of (+)-ethyl 12bα-methyl-1,2,3,4,6,7,12,12b-octahydroindolo[2,3-a]quinolizine-1α-carboxylate with lithium aluminium hydride, following

the procedure and using the reactants in the quantities described in Example 1.

Yield: 6.08 g. (99 %) of an oily product crystallizing upon standing

Melting point: 185 ° to 187 °C

$[\alpha]_D^{22}$ = -17.2° (C=2.0; ethanol)

$R_f$=0.26 (Polygram SIL G/$UV_{254}$, ethyl acetate containing 10 % of triethyl amine, u.v. light)

Example 6

(-)-1α- Hydroxymethyl.-12bα-methyl-1,2,3,4,6,7,12,12b-octahydroindolo[2,3-a]quinolizine ethanesulfonate

To a suspension of 6.00 g. (0.0222 mole) of the base prepared according to Example 5 in 10 ml. of acetone a solution of 2.44 g. (0.0222 mole) of ethanesulfonic acid in 5 ml. of isopropyl alcohol is added and the mixture is diluted with 5 ml. of acetone. The precipitated solid is dissolved by heating, and the solution is in portions diluted with 185 ml. of acetone to yield the title compound in a crystalline form.

Yield: 6.27 g. (74 %)

Melting point: 185 ° to 188 °C (the product solidifies and remelts at 238 ° to 239 °C)

$[\alpha]_D^{22}$ = -15.0° (c=2.0; ethanol)

Analysis for $C_{19}H_{28}N_2O_4S$ (380.49):

| | | | | |
|---|---|---|---|---|
| calculated: | C 59.97 %, | H 7.42 %, | N 7.36 %, | S 8.43 %; |
| found: | C 59.97 %, | H 7.74 %, | N 7.34 %, | S 8.33 %. |

Example 7

(±)-1β- Hydroxymethyl -12bα-methyl-1,2,3,4,6,7,12,12b-octahydroindolo[2,3-a]quinolizine

A solution of 0.624 g. (0.002 mole) of (±)-ethyl 12bα-methyl-1,2,3,4,6,7,12,12b-octahydroindolo[2,3-a]quinolizine-1β-carboxylate in 3 ml. of dry tetrahydrofurane is added dropwise to a suspension of 0.600 g. (0.016 mole) of lithium aluminium hydride in 20 ml. of dry tetrahydrofuran under an argon atmosphere, at room temperature with stirring, in 10 minutes. The mixture is stirred for a further one hour, whereupon 0.6 ml. of water, 0.6 ml. of a 15 % aqueous sodium hydroxide solution and subsequently 1.8 ml. of water are added carefully, dropwise, under ice cooling and vigorous stirring. The precipitate is filtered off, washed with three 2-ml. portions of tetrahydrofurane, the combined tetrahydrofurane solution is dried over solid anhydrous magnesium sulfate and evaporated. The residue (0.445 g.) is purified by column chromatography on silica gel (40 g., Geduran Si 60, 0.063 to 0.200 mm.) using ethyl acetate containing 10 % of triethylamine for the elution and subsequent recrystallization from 5 ml. of 1,2-dichloroethane.

Yield: 0.180 g. (33 %)

Melting point: 209 °to 211 °C

$R_f$=0.45 (Polygram SIL G/$UV_{254}$, ethyl acetate containing 10 % of triethyl amine, u.v. light)

Example 8

(±)-1β- Hydroxymethyl -12bα-methyl-1,2,3,4,6,7,12,12b-octahydroindolo[2,3-a]quinolizine ethanesulfonate

To a suspension of 1.50 g. (0.00555 mole) of the base prepared according to Example 7 in 25 ml. of isopropyl alcohol a solution of 0.611 g. (0.00555 mole) of ethanesulfonic acid in 5 ml. of isopropyl alcohol is added, the base is dissolved by heating and the solution is evaporated on a rotating evaporator. The foamy residue is dissolved in 3 ml. of isopropyl alcohol and the solution is diluted with 150 ml. of acetone. The product becomes crystalline slowly, upon rubbing.

Yield: 1.60 g. (76 %)

Melting point: 189 ° to 193 °C

Analysis for $C_{19}H_{28}N_2O_4S$ (380.49):

calculated: C 59.97 %, H 7.42 %, N 7.36 %, S 8.43 %;

found: C 59.84 %, H 7.70 %, N 7.31 %, S 8.25 %.

Example 9

(±)-1β- Hydroxymethyl -12,12bβ-dimethyl-1,2,3,4,6,7,12,12b-octahydroindolo[2,3-a]quinolizine

A solution of 0.420 g. (0.00128 mole) of (±)-ethyl 12,12bβ-dimethyl-1,2,3,4,6,7,12,12b-octahydroindolo[2,3-a]quinolizine-1β-carboxylate in 30 ml. of dry tetrahydrofuran is added dropwise to a suspension of 0.380 g. (0.01 mole) of lithium aluminium hydride in 20 ml. of dry tetrahydrofuran under an argon atmosphere, at room temperature in ten minutes, and the mixture is

stirred for a further one hour at room temperature. Under ice cooling and stirring, 0.5 ml. of water and 0.5 ml. of a 15 % aqueous sodium hydroxide solution are carefully added followed by a dropwise addition of 1.5 ml. of water. The precipitate is filtered off, washed with tetrahydrofurane and the solution is evaporated. The solid residue is purified by column chromatography on silica gel (40 g. of Geduran Si 60, 0.063 to 0.200 mm.) using ethyl acetate containing 10 % of diethylamine as an eluent.

Yield: 0.285 g. (78 %) of the title compound, which can be further purified by crystallization from ethyl acetate.

Melting point: 170 ° to 174 °C

$R_f$: 0.35 (Polygram SIL G/$UV_{254}$, ethyl acetate containing 10 % of diethyl amine, u.v. light)

Example 10

(±)-1β-Ethyl-1α- hydroxymethyl -12bβ-methyl-1,2,3,4,6,7,12,12b-octahydroindolo[2,3-a]quinolizine

A solution of 0.075 g. (0.00022 mole) of (±)-ethyl 1β-ethyl-12bβ-methyl-1,2,3,4,6,7,12,12b-octahydroindolo-[2,3-a]quinolizine-1α-carboxylate in 0.6 ml. of dry tetrahydrofurane is added dropwise to a suspension of 0.084 g. (0.0022 mole) of lithium aluminium hydride in 2 ml. of dry tetrahydrofurane at room temperature, with stirring, under an argon atmosphere, and the mixture is stirred for a further one hour. The excess reducing agent is decomposed by the addition of 0.1 ml.

of water, 0.1 ml. of a 15 % aqueous sodium hydroxide solution and 0.3 ml. of water dropwise, the aluminium hydroxide precipitate is filtered off and washed with three 1-ml. portions of tetrahydrofurane. The combined tetrahydrofurane solution is dried over solid anhydrous magnesium sulfate and evaporated to yield the pure title compound.

Yield: 0.064 g. (97 %) of a foamy material

$R_f$: 0.32 (Polygram SIL G/$UV_{254}$, tetrachloromethane containing 5 % of diethyl amine, u.v. light)

Example 11

(±)-1α- Hydroxymethyl -1β,12,12bβ-trimethyl-1,2,3,4,6,7,12,12b-octahydroindolo[2,3-a]-quinolizine

A solution of 0.340 g. (0.001 mole) of (±)-ethyl 1β,12,12bβ-trimethyl-1,2,3,4,6,7,12,12b-octahydroindolo[2,3-a]quinolizine-1α-carboxylate in 20 ml. of dry tetrahydrofurane is added dropwise to a suspension of 0.300 g. (0.00789 mole) of lithium aluminium hydride in 30 ml. of dry tetrahydrofurane under an argon atmosphere, at room temperature, in 10 minutes, and the mixture is stirred at room temperature for a further 4 hours. Under ice cooling and stirring 0.3 ml. of water and subsequently 0.3 ml. of a 15 % aqueous sodium hydroxide solution are carefully added to the mixture, followed by a dropwise addition of 1.2 ml. of water. The precipitate is filtered off, washed with tetrahydrofurane, the combined tetrahydrofurane solution is dried over solid anhydrous magnesium sulfate and evaporated.

Yield: 0.197 g. (66 %)

Melting point: 131 to 134 °C

$R_f$: 0.69 (Polygram SIL G/$UV_{254}$, ethyl acetate containing 1 % of diethyl amine, u.v. light)

<u>Example 12</u>

A- and B-epimers of (±)-1β-(1-hydroxyethyl)-12bβ-methyl-1,2,3,4,6,7,12,12b-octahydroindolo[2,3-a]-quinolizine

0.266 g. (0.007 mole) of sodium borohydride is added to a solution of 0.198 g. (0.0007 mole) of 1β-acetyl-12bβ-methyl-1,2,3,4,6,7,12,12b-octahydro-indolo[2,3-a]quinolizine in 10 ml. of dry methanol in small portions, within 10 minutes, and the mixture is allowed to stand at room temperature for two hours. After the addition of 5 ml. of acetone the solution is allowed to stand for an additional 10 minutes, whereupon it is evaporated and 5 ml. of acetone are distilled off. To the residue there are added 10 ml. of water and 10 ml. of dichloromethane, the mixture is thoroughly shaken and the phases are separated. The aqueous part is washed with two 10-ml. portions of dichloromethane, dried over anhydrous magnesium sulfate and evaporated. The epimeric mixture obtained is separated into its components by chromatography on 40 g. of silica gel (Geduran Si 60, 0.063-0.200 mm.) using toluene containing 10 % of diethylamine as eluant.

A-epimer:

Yield: 0.090 g. (45 %) of a foamy material

$R_f$: 0.43 (Polygram SIL G/$UV_{254}$, toluene containing 10 % of diethyl amine, u.v. light)

B-epimer:

Yield: 0.107 g. (54 %) of a foamy material

$R_f$: 0.28 (Polygram SIL G/$UV_{254}$, toluene containing 10 % of diethyl amine, u.v. light)

Example 13

A- and B-epimers of (±)-1β-(1-hydroxyethyl)-12bβ-methyl-1,2,3,4,6,7,12,12b-octahydroindolo/2,3-a/quinolizine

0.099 g. (0.00035 mole) of 1β-acetyl-12bβ-methyl-1,2,3,4,6,7,12,12b-octahydroindolo/2,3-a/quinolizine are hydrogenated in 5 ml. of ethanol, in the presence of 1 g. of Raney nickel catalyst, at room temperature, under atmospheric pressure for 25 hours. The catalyst is filtered off, the solution is evaporated and the residue is separated into its components by chromatography on 40 g. of silica gel (Geduran Si 60, 0.063-0.200 mm.) using toluene containing 10 % of diethylamine as an eluent.

A-epimer:

Yield: 0.015 g. (15 %)

$R_f$: 0.43 (Polygram SIL G/$UV_{254}$, toluene containing 10 % of diethyl amine, u.v. light)

B-epimer:

Yield: 0.082 g. (82 %)

$R_f$: 0.28 (Polygram SIL G/$UV_{254}$, toluene containing 10 % of diethyl amine, u.v. light)

Example 14

(±)-9-Bromo-1β-hydroxymethyl-12bβ-methyl-1,2,3,4,6,7,12,12b-octahydroindolo[2,3-a]-quinolizine

0.104 g. (0.0031 mole) of sodium borohydride are dissolved in 1 ml. of dry diethylene glycol dimethyl ether under an argon atmosphere, with stirring and after stirring for several minutes 0.391 g. (0.001 mole) of (±)-ethyl 9-bromo-12bβ-methyl-1,2,3,4,6,7,12,12b-octahydroindolo[2,3-a]quinolizine-1β-carboxylate are added to the solution. Thereafter 0.140 g. (0.00105 mole) of anhydrous aluminium(III) chloride are added to the mixture under vigorous stirring, which is then stirred at room temperature for one hour and at 100 °C for an additional one hour. The mixture is allowed to cool to room temperature. It is then poured onto a mixture of 5 g. of broken ice and 0.7 ml. of concentrated hydrochloric acid, the mixture is rendered alkaline with a 10 % aqueous sodium carbonate solution, extracted with chloroform, dried over solid anhydrous magnesium sulfate and evaporated. The crude product is purified by chromatography on 30 g. of silica gel (Geduran Si 60, 0.063-0.200 mm.) using toluene containing 10 % of diethylamine for the elution.

Yield: 0.180 g. (52 %)

Melting point: 194 ° to 196 °C

$R_f$: 0.27 (Polygram SIL G/$UV_{254}$, ethyl acetate containing 5 % of triethyl amine, u.v. light)

Example 15

(±)-10-Bromo-1β-hydroxymethyl-12bβ-methyl-1,2,3,4,6,7,12,12b-octahydroindolo/2,3-a7quinolizine

Following the procedure described in Example 14 but starting from 0.391 g. (0.001 mole) of (±)-ethyl 10-bromo-12bβ-methyl-1,2,3,4,6,7,12,12b-octahydroindolo/2,3-a7quinolizine-1β-carboxylate, and purifying the crude product obtained on 30 g. of silica gel (Geduran Si 60, 0.063-0.200 mm.) using toluene containing 20 % of diethylamine as eluant, the title compound is obtained.

Yield: 0.230 g. (66 %)

Melting point: 218 ° to 220 °C

$R_f$: 0.18 (Polygram SIL G/$UV_{254}$, toluene containing 20 % of diethyl amine, u.v. light)

Example 16

(±)-8,9-Dibromo-1β-hydroxymethyl-12bβ-methyl-1,2,3,4,6,7,12,12b-octahydroindolo/2,3-a7-quinolizine

Following the procedure described in Example 14 but starting from 0.130 g. (0.000276 mole) of (±)-ethyl 8,9-dibromo-12bβ-methyl-1,2,3,4,6,7,12,12b-octahydroindolo/2,3-a7quinolizine-1β-carboxylate and purifying the crude product on 20 g. of silica gel (Geduran Si 60, 0.063-0.200 mm.) using toluene containing 20 % of diethyl amine as eluant, the title compound is obtained.

Yield: 0.054 g. (46 %)

$R_f$: 0.27 (Polygram SIL G/$UV_{254}$, toluene containing 20 % of diethyl amine, u.v. light)

Example 17

(±)-1β-Hydroxymethyl-12bβ-methyl-8-nitro-1,2,3,4,6,7,12,12b-octahydroindolo/2,3-a7quinolizine

Following the procedure described in Example 14 but starting from 0.357 g. (0.001 mole) of (±)-ethyl 12bβ-methyl-8-nitro-1,2,3,4,6,7,12,12b-octahydroindolo-/2,3-a7quinolizine-1β-carboxylate and purifying the crude product obtained by chromatography on 30 g. of silica gel (Geduran Si 60, 0.063-0.200 mm.) using toluene containing 20 % of diethylamine as eluant, the title compound is obtained.

Yield: 0.080 g. (25 %)

Melting point: 246 ° to 248 °C

$R_f$:0.2 (Polygram SIL G/$UV_{254}$, toluene containing 20 % of diethyl amine, u.v. light)

Example 18

(±)-1β-Hydroxymethyl-12bβ-methyl-10-nitro-1,2,3,4,6,7,12,12b-octahydroindolo/2,3-a7-quinolizine

Following the procedure described in Example 14 but starting from 0.357 g. (0.001 mole) of (±)-ethyl 12bβ-methyl-10-nitro-1,2,3,4,6,7,12,12b-octahydroindolo-/2,3-a7quinolizine-1β-carboxylate and purifying the crude product obtained by chromatography on 30 g. of silica gel (Geduran Si 60, 0.063-0.200 mm.) using

toluene containing 10 % of diethylamine as eluant, the title compound is obtained.

Yield: 0.165 g. (52 %)

Melting point: 196 ° to 198 °C

$R_f$: 0.17 (Polygram SIL G/$UV_{254}$, toluene containing 10 % of diethyl amine, u.v. light)

Example 19

(±)-10-Amino-1β-hydroxymethyl-12bβ-methyl-1,2,3,4,6,7,12,12b-octahydroindolo/2,3-a/quinolizine

1.40 g. (0.0105 mole) of anhydrous aluminium(III) chloride are dissolved in 50 ml. of dry ether under stirring and ice cooling, and the solution is added dropwise to a suspension of 0.4 g. (0.0105 mole) of lithium aluminium hydride in 50 ml. of dry ether, under stirring and ice cooling, in an argon atmosphere. Thereafter, the suspension is boiled with stirring so that the refluxing ether should dissolve from an extractor 0.357 g. (0.001 mole) of (±)-ethyl 12bβ-methyl-10-nitro-1,2,3,4,6,7,12,12b-octahydro-indolo/2,3-a/quinolizine-1β-carboxylate. When the dissolution is complete (about 10 minutes) the mixture is refluxed for a further five hours, whereupon 25 ml. of water are added under cooling and stirring followed by the dissolution of 4.0 g. of sodium hydroxide. After stirring for 30 additional minutes the aqueous phase is washed with four 25-ml. portions of ether, the combined organic phase is dried over solid anhydrous

magnesium sulfate and evaporated. The crude product is purified by chromatography on 30 g. of silica gel (Geduran Si 60, 0.063-0.200 mm.) using toluene containing 30 % of diethylamine as eluant.

Yield: 0.13 g. (46 %)

$R_f$: 0.17 (Polygram SIL G/$UV_{254}$, toluene containing 30 % of diethyl amine, u.v. light)

Example 20

(±)-10-Amino-1β-hydroxymethyl-12bβ-methyl-1,2,3,4,6,7,12,12b-octahydroindolo[2,3-a]-quinolizine

0.10 g. (0.0003 mole) of (±)-1β-hydroxymethyl-12bβ-methyl-10-nitro-1,2,3,4,6,7,12,12b-octahydro-indolo[2,3-a]quinolizine is dissolved in 10 ml. of dry ethanol (for the preparation see Example 18) and the solution is hydrogenated in the presence of 0.2 g. of a 10 % palladium-on-charcoal catalyst. When the hydrogen uptake ceases, the catalyst is filtered off, washed with two 2-ml. portions of ethanol and the product is isolated by evaporation on a rotating evaporator.

Yield: 0.09 g. (99 %)

$R_f$: 0.17 (Polygram SIL G/$UV_{254}$, toluene containing 30 % of diethyl amine, u.v. light)

Example 21

(±)-1β-Hydroxymethyl-12-benzyl-12bβ-methyl-1,2,3,4,6,7,12,12b-octahydroindolo[2,3-a]-quinolizine

A solution of 0.04 g. (0.0001 mole) of (±)-ethyl 12-benzyl-12bα-methyl-1,2,3,4,6,7,12,12b-octahydroindolo/2,3-a7quinolizine-1-carboxylate in 3 ml. of dry tetrahydrofurane is added dropwise to a suspension of 0.038 g. (0.001 mole) of lithium aluminium hydride in 2 ml. of dry tetrahydrofurane under an argon atmosphere, at room temperature, and the mixture is stirred at room temperature for one hour. 0.05 ml. of water, 0.05 ml. of a 15 % aqueous sodium hydroxide solution and subsequently 0.15 ml. of water are added to the reaction mixture carefully, dropwise, under ice cooling and stirring. The precipitate is filtered off, washed with tetrahydrofurane and the solution is evaporated. The solid residue is purified by chromatography on 10 g. of silica gel (Geduran Si 60, 0.063-0.200 mm.) using toluene containing 30 % of diethylamine as eluant.

Yield: 0.012 g. (33.3 %) of an yellow oil

$R_f$: 0.60 (Polygram SIL G/$UV_{254}$, toluene containing 30 % of diethyl amine, u.v. light)

Example 22

(±)-1β-Hydroxymethyl-9-chloro-12bβ-methyl-1,2,3,4,6,7,12,12b-octahydroindolo/2,3-a7-quinolizine

0.378 g. (0.0112 mole) of sodium borohydride are dissolved in 18 ml. of dry diethyleneglycol dimethyl ether under an argon atmosphere, with stirring.

After stirring for several minutes 2.08 g. (0.006 mole) of (±)-ethyl 9-chloro-12bβ-methyl-1,2,3,4,6,7,12,12b-octahydroindolo[2,3-a]quinolizine-1β-carboxylate are added to the solution. Thereafter, 0.504 g. (0.00378 mole) of anhydrous aluminium(III) chloride are added to the mixture under vigorous stirring, and the mixture is stirred at room temperature for one hour and at 100 °C for a further one hour. The mixture is allowed to cool to room temperature and is then poured onto a mixture of 15 g. of broken ice and 2.25 ml. of concentrated hydrochloric acid. The mixture is rendered alkaline with a 10 % aqueous sodium carbonate solution, extracted with chloroform, dried over solid anhydrous magnesium sulfate and is then evaporated. The crude product obtained is purified by chromatography on 150 g. of silica gel (Geduran Si 60, 0.063-0.200 mm.) using toluene containing 20 % of diethylamine as eluant.

Yield: 1.5 g. (82 %) of the title compound, which can be further purified by crystallization from acetone.

Melting point: 200-202 °C

$R_f$: 0.38 (Polygram SIL G/$UV_{254}$, toluene containing 20 % of diethyl amine, u.v. light).

Example 23

(±)-1β-Hydroxymethyl-9-chloro-12bβ-methyl-1,2,3,4,6,7,12,12b-octahydroindolo[2,3-a]-quinolizine ethanesulfonate

1.2 g. (0.0039 mole) of the base prepared

according to Example 22 are dissolved in 50 ml. of chloroform, whereupon 0.429 g. (0.0039 mole) of ethanesulfonic acid are added to the solution, under stirring and ice cooling. The crude product obtained by evaporation of the solvent is recrystallized from isopropyl alcohol.

Yield: 1.06 g. (65 %)

Melting point: 160 ° to 163 °C

Analysis for $C_{19}H_{27}ClN_2O_4S$ (414.95 ):

calculated: C 54.99 %, H 6.56 %, N 6.75 %, $Cl_{tot.}$ 8.54 %, S 7.73 %;

found: C 55.10 %, H 6.71 %, N 6.75 %, $Cl_{tot.}$ 8.64 %, S 7.69 %.

Example 24

(±)-9-Hydroxy-1β-hydroxymethyl-12bβ-methyl-1,2,3,4,6,7,12,12b-octahydroindolo[2,3-a]quinolizine

A solution of 0.31 g. (0.00094 mole) of (±)-ethyl 9-hydroxy-12bβ-methyl-1,2,3,4,6,7,12,12b-octahydroindolo[2,3-a]quinolizine-1β-carboxylate in 20 ml. of dry tetrahydrofurane is added dropwise to a suspension of 0.38 g. (0.01 mole) of lithium aluminium hydride in 20 ml. of dry tetrahydrofurane, under an argon atmosphere, at room temperature, in 5 minutes, whereupon the reaction mixture is stirred at room temperature for 30 minutes. 0.4 ml. of water, 0.4 ml. of a 15 % aqueous sodium hydroxide solution and subsequently 1.2 ml. of water are added dropwise to the reaction mixture, under ice cooling and stirring. The precipitate is

filtered off, washed with tetrahydrofurane and the solution is evaporated. The solid residue is purified by chromatography on 40 g. of silica gel (Geduran Si 60, 0.063-0.200 mm.) using toluene containing 20 % of diethylamine as eluant.

Yield: 0.038 g. (14.1 %) of a pale yellow oil

$R_f$: 0.31 (Polygram SIL G/$UV_{254}$, toluene containing 20 % of diethyl amine, u.v. light)

Example 25

(±)-12α-Phenyl-1β-hydroxymethyl-1,2,3,4,6,7,12,12b-octahydroindolo/2,3-a7quinolizine

A solution of 0.442 g. (0.00118 mole) of (±)-ethyl 12α-phenyl-1,2,3,4,6,7,12,12b-octahydroindolo/2,3-a7-quinolizine-1β-carboxylate in 4 ml. of dry tetrahydrofurane is added dropwise to a suspension of 0.46 g. (0.012 mole) of lithium aluminium hydride in 10 ml. of dry tetrahydrofurane, under an argon atmosphere and stirring. at room temperature in 5 minutes. After stirring for an additional hour 0.5 ml. of water, 0.5 ml. of a 15 % aqueous sodium hydroxide solution and subsequently 1.5 ml. of water are carefully added to the mixture, under ice cooling. The precipitate is filtered off, washed with three 5-ml. portions of tetrahydrofurane, the combined tetrahydrofurane extract is dried over solid anhydrous magnesium sulfate and evaporated. The crude product is purified by column chromatography on 50 g. of silica gel (Geduran Si 60, 0.063-0.200 mm.) using toluene containing 5 % of diethylamine for the elution.

Yield: 0.120 g. (31 %) of the title compound crystallizing upon standing

Melting point: 243 ° to 247 °C

$R_f$: 0.45 (Polygram SIL G/$UV_{254}$, toluene containing 5 % of diethyl amine, u.v. light).

Example 26

A- and B-epimers of (±)-1β-(phenyl-hydroxymethyl)-12bβ-methyl-1,2,3,4,6,7,12,12b-octahydroindolo-[2,3-a]quinolizine

Into a solution of 0.955 g. (0.0028 mole) of (±)-1β-benzoyl-12bβ-methyl-1,2,3,4,6,7,12,12b-octahydro-indolo[2,3-a]quinolizine in 50 ml. of dry methanol 1.60 g. (0.042 mole) of sodium borohydride are added at room temperature, with stirring, in small portions in 3 hours. After stirring for one additional hour, 25 ml. of acetone are added to the mixture, which is then stirred for 30 minutes, evaporated and 25 ml. of acetone are distilled off. The residue is diluted with 50 ml. of water and 50 ml. of dichloromethane and is thoroughly shaken. The phases are separated. The aqueous phase is washed with two 10-ml. portions of dichloromethane, and the combined dichloromethane solution is dried over solid anhydrous magnesium sulfate and evaporated. The epimeric mixture obtained is separated into its components by chromatography on 200 g. of silica gel (Geduran Si 60, 0.063-0.200 mm.) using toluene containing 10 % of diethylamine as eluant.

A-epimer:

Yield: 0.658 g. (68 %) The product can be crystallized from chloroform.

Melting point: 212 ° to 214 °C

$R_f$:0.62 (Polygram SIL G/$UV_{254}$, toluene containing 10 % of diethyl amine, u.v. light)

B-epimer:

Yield: 0.197 g (20 %) of a thick oil solidifying upon standing

$R_f$: 0.31 (Polygram SIL G/$UV_{254}$, toluene containing 10 % of diethyl amine, u.v. light).

Example 27

Ethanesulfonate of the A-epimer of (±)-1β-(phenyl-hydroxymethyl)-12bβ-methyl-1,2,3,4,6,7,12,12b-octahydroindolo[2,3-a]quinolizine

0.623 g. (0.0018 mole) of the A-epimer base prepared according to Example 26 are dissolved in a warm mixture of 15 ml. of acetone and 5 ml. of isopropyl alcohol, whereupon a solution of 0.209 g. (0.0019 mole) of ethanesulfonic acid in 2.5 ml. of isopropyl alcohol is added dropwise, with stirring, in 5 minutes. After standing for a short time the product separates out in a crystalline form.

Yield: 0.685 g. (83 %)

Melting point: 275 ° to 276 °C (decomp.)

Analysis for $C_{25}H_{32}N_2O_4S$ (456.58):

calculated: C 65.76 %, H 7.06 %, N 6.14 %, S 7.02 %;

found: C 65.87 %, H 7.12 %, N 6.14 %, S 6.98 %.

Example 28

(±)-1β-Hydroxymethyl-12bβ-methyl-9-methoxy-1,2,3,4,6,7,12,12b-octahydroindolo[2,3-a]quinolizine

A solution of 0.747 g. (0.00218 mole) of (±)-ethyl 12bβ-methyl-9-methoxy-1,2,3,4,6,7,12,12b-octahydroindolo-

/2,3-a7quinolizine-11-carboxylate in 7 ml. of dry tetrahydrofurane is added dropwise, under an argon atmosphere, in 10 minutes to a stirred suspension of 0.663 g. (0.0175 mole) of lithium aluminium hydride in 15 ml. of dry tetrahydrofurane, at room temperature. The reaction mixture is stirred for an additional 80 minutes, whereupon 0.8 ml. of water and 0.8 ml. of a 15 % aqueous sodium hydroxide solution are carefully added under ice cooling, followed by the dropwise addition of 2.4 ml. of water. The precipitate is filtered off, washed with three 2-ml. portions of tetrahydrofurane, the combined tetrahydrofurane solution is evaporated and 10 ml. of dry ethanol are distilled off. The crude product is purified by column chromatography on 80 g. of silica gel (Geduran Si 60, 0.063-0.200 mm.) using cyclohexane containing 10 % of diethyl amine and 20 % of isopropyl alcohol as eluant.

Yield: 0.492 g. (75 %) of the title compound, which can be crystallized from isopropyl alcohol.

Melting point: 189 $^{\circ}$ to 191 $^{\circ}$C

$R_f$: 0.43 (Polygram SIL G/$UV_{254}$, cyclohexane containing 10 % of diethyl amine and 20 % of isopropyl alcohol, u.v. light).

Example 29

(±)-1β-Hydroxymethyl-12bβ-methyl-9-methoxy-1,2,3,4,6,7,12,12b-octahydroindolo[2,3-a]-quinolizine ethanesulfonate

To a suspension of 0.454 g. (0.0015 mole) of the base prepared according to Example 28 in 3 ml. of isopropyl alcohol a solution of 0.176 g. (0.0016 mole) of ethanesulfonic acid in 1 ml. of isopropyl alcohol is added. The base is dissolved under stirring, the mixture is evaporated and the residue is dissolved in 3 ml. of 1,2-dichloroethane. The product is obtained in a crystalline form after standing overnight.

Yield: 0.466 g. (76 %)

Melting point: 177 ° to 181 °C

Analysis for $C_{20}H_{30}N_2O_5S$:

calculated: C 58.51 %, H 7.37 %, N 6.82 %, S 7.81 %;

found: C 58.47 %, H 7.29 %, N 6.78 %, S 7.80 %.

Example 30

(±)-1β-hydroxymethyl-12bα-methyl-9-methoxy-1,2,3,4,6,7,12,12b-octahydroindolo[2,3-a]quinolizine

A solution of 0.183 g. (0.000525 mole) of (±)-ethyl 12bα-methyl-9-methoxy-1,2,3,4,6,7,12,12b-octahydroindolo[2,3-a]quinolizine-1β-carboxylate in 2 ml. of dry tetrahydrofurane is added dropwise, in 10 minutes, under an argon atmosphere to a suspension of 0.163 g. (0.00428 mole) of lithium aluminium hydride in 4 ml. dry tetrahydrofurane, while stirring the mixture. After stirring for an additional 80 minutes,

0.2 ml. of water and 0.2 ml. of a 15 % aqueous sodium hydroxide solution are added dropwise to the reaction mixture under ice cooling, followed by the dropwise addition of 0.6 ml. of water. The precipitate is filtered off, washed with three 1-ml. portions of tetrahydrofurane, the combined tetrahydrofurane solution is evaporated and 5 ml. of dry ethanol are distilled off. The crude product is purified by column chromatography on 20 g. of silica gel (Geduran Si 60, 0.063-0.200 mm.) using cyclohexane containing 10 % of diethyl amine and 20 % of isopropyl alcohol as eluant.

Yield: 0.117 g. (73 %) of a foamy material

Rf: 0.57 (Polygram SIL G/$UV_{254}$, cyclohexane containing 10 % of diethyl amine and 20 % of isopropyl alcohol, u.v. light).

Example 31

(±)-9,12bα-Dimethyl-1β-hydroxymethyl-1,2,3,4,6,7,12,12b-octahydroindolo[2,3-a]quinolizine

A solution of 1.956 g. (0.006 mole) of (±)-ethyl 9,12bα-dimethyl-1,2,3,4,6,7,12,12b-octahydroindolo-[2,3-a]quinolizine-1β-carboxylate in 15 ml. of dry tetrahydrofurane are added dropwise, in 20 minutes, under an argon atmosphere and stirring to a suspension of 1.824 g. (0.048 mole) of lithium aluminium hydride in 50 ml. of dry tetrahydrofurane, at room temperature. After stirring for two additional hours, 1.8 ml. of water and 1.8 ml. of a 15 % aqueous sodium hydroxide solution are added dropwise to the cooled solution, followed by dropwise addition of 5.4 ml. of water. The precipitate is filtered off, washed with three

10-ml. portions of tetrahydrofuran, and the combined tetrahydrofuran solution is dried over solid anhydrous magnesium sulfate and evaporated. The crude product is purified by chromatography on 200 g. of silica gel (Geduran Si 60, 0.063-0.200 mm.) using cyclohexane containing 10 % of diethylamine and 20 % of ethanol as eluant.

Yield: 1.175 g. (69 %) of the title compound, crystallizing upon standing

Melting point: 173 ° to 176 °C

$R_f$: 0.55 (Polygram SIL G/$UV_{254}$, cyclohexane containing 10 % of diethylamine and 20 % of ethanol, u.v. light).

Example 32

(±)-9,12bβ-Dimethyl-1α-hydroxymethyl-1,2,3,4,6,7,12,12b-octahydroindolo[2,3-a]quinolizine ethanesulfonate

A solution of 0.408 g. (0.0037 mole) of ethanesulfonic acid in 2 ml. of isopropyl alcohol are added dropwise, under stirring, in 5 minutes to a warm suspension of 0.989 g. (0.0035 mole) of the base prepared according to Example 31 in 7 ml. of isopropyl alcohol. The base is dissolved by stirring, the solution is evaporated and the residue is dissolved in 10 ml. of 1,2-dichloroethane. The mixture is allowed to stand overnight to yield the title compound in a crystalline form.

Yield: 1.220 g. (88 %)

Melting point: 167 ° to 170 °C

Analysis for $C_{20}H_{30}N_2O_4S$ (394.52):

calculated: C 60.88 %, H 7.67 %, N 7.10 %, S 8.13 %;

found: C 60.97 %, H 7.81 %, N 7.12 %, S 8.10 %.

Example 33

(±)-1β-Hydroxymethyl-12bβ-methyl-10-methoxy-1,2,3,4,6,7,12,12b-octahydroindolo[2,3-a]-quinolizine

The title compound is prepared from 0.075 g. (0.00022 mole) of (±)-ethyl 12bβ-methyl-10-methoxy-1,2,3,4,6,7,12,12b-octahydroindolo[2,3-a]quinolizine-1β-carboxylate following the procedure and using the reactants in the quantities described in Example 10. The crude product obtained is purified by chromatography on 20 g. of silica gel (Geduran Si 60, 0.063-0.200), using cyclohexane containing 10 % of diethylamine and 20 % of ethanol for the elution.

Yield: 0.038 g. (58 %) of a thick oil

$R_f$: 0.49 (Polygram SIL G/$UV_{254}$, cyclohexane containing 10 % of diethylamine and 20 % of ethanol, u.v. light).

Example 34

(±)-1β-Butyl-12,12bβ-dimethyl-3α-hydroxymethyl-1,2,3,4,6,7,12,12b-octahydroindolo[2,3-a]-quinolizine

A solution of 0.100 g. (0.00026 mole) of (±)-ethyl 1β-butyl-12,12bβ-dimethyl-1,2,3,4,6,7,12,12b-octahydroindolo[2,3-a]quinolizine-1α-carboxylate in

5 ml. of dry tetrahydrofuran is added dropwise, under argon atmosphere and stirring, to a suspension of 0.2000 g. (0.0053 mole) of lithium aluminium hydride in 5 ml. of dry tetrahydrofurane. During the addition the mixture is kept at boiling temperature. It is then refluxed for 8 hours. The excess reducing agent is decomposed by the succesive, dropwise addition of 0.2 ml. of water, 0.2 ml. of a 15 % aqueous sodium hydroxide solution and 0.6 ml. of water, under ice cooling. The precipitate is filtered off and washed with tetrahydrofurane. The combined tetrahydrofuran solution is dried over solid anhydrous magnesium sulfate and evaporated. The residue is purified by chromatography on 10 g. of silica gel (Geduran Si 60, 0.063-0.200 mm.) using n-hexane containing 25 % of diethylamine as eluant.

Yield: 0.033 g. (36.6 %) of an oily product solidifying upon standing

Melting point: 123 ° to 125 °C

$R_f$: 0.55 (Polygram SIL G/$UV_{254}$, n-hexane containing 25 % of diethylamine, u.v. light).

Example 35

(±)-1β-Benzyl-12,12bβ-dimethyl-1α-hydroxymethyl-1,2,3,4,6,7,12,12b-octahydroindolo/2,3-a/-quinolizine

A solution of 0.100 g. (0.00024 mole) of (±)-ethyl 1β-benzyl-12,12bβ-dimethyl-1,2,3,4,6,7,12,12b-octahydro-

indolo/2,3-a/quinolizine-1α-carboxylate in 15 ml. of dry tetrahydrofurane is added dropwise, under an argon atmosphere and stirring to a suspension of 0.200 g. (0.0053 mole) of lithium aluminium hydride in 15 ml. of dry tetrahydrofuran. During the addition the mixture is kept at boiling temperature. The mixture is then refluxed for 8 hours, while two further 0.200 g. portions of lithium aluminium hydride are added. Thereafter the excess reducing agent is decomposed by the successive, dropwise addition of 0.6 ml. of water, 0.6 ml. of a 15 % aqueous sodium hydroxide solution and 2 ml. of water, under stirring and ice cooling. The precipitate is filtered off and washed with tetrahydrofuran. The combined tetrahydrofurane solution is dried over solid anhydrous magnesium sulfate and evaporated. The residue is purified by chromatography on 10 g. of silica gel (Geduran Si 60, 0.063-0.200 mm.) using a 1:1 mixture of petroleum ether containing 2 % of diethylamine and ethyl acetate as eluant.

Yield: 0.023 g. (25.6 %) of an oily product solidifying upon standing

Melting point: 185 $^{\circ}$ to 190 $^{\circ}C$

Rf: 0.45 (Polygram SIL G/$UV_{254}$, 1:1 mixture of petroleum ether containing 2 % of diethyl amine and ethyl acetate, u.v. light).

Example 36

(±)-1β-(1-Hydroxy-3-phenylpropyl)-12bα-(2-phenylethyl)-1,2,3,4,6,7,12,12b-octahydroindolo-[2,3-a]quinolizine

To a solution of 0.008 g. (0.000017 mole) of (±)-1β-(3-phenylpropionyl)-12bα-(2-phenylethyl)-1,2,3,4,6,7,12,12b-octahydroindolo[2,3-a]quinolizine in 1 ml. of dry methanol 0.032 g. (0.00084 mole) of sodium borohydride are added in portions, under stirring. After standing for one hour 2 ml. of acetone are added to the mixture, which is then stirred for 30 minutes at room temperature, evaporated and a further 2-ml. portion of acetone is distilled off. The residue is supplemented with 3 ml. of water and extracted with three 3-ml. portions of dichloromethane. The dichloromethane solution is dried over solid anhydrous magnesium sulfate, evaporated and the residue is purified by column chromatography on 10 g. of silica gel (Geduran Si 60, 0.063-0.200 mm.) using cyclohexane containing 10 % of diethylamine as aluant.

Yield: 0.005 g. (62 %) of an oily product crystallizing upon standing.

Melting point: 158 ° to 163 °C

$R_f$: 0.21 (Polygram SIL G/UV$_{254}$, cyclohexane containing 10 % of diethylamine, u.v. light).

Example 37

(±)-1β-(1-Hydroxy-3-phenylpropyl)-12bβ-(2-phenylethyl)-1,2,3,4,6,7,12,12b-octahydroindolo[2,3-a]-quinolizine

To a solution of 0.012 g. (0.000026 mole) of (±)-1α-(3-phenylpropionyl)-12bα-(2-phenylethyl)-1,2,3,4,6,7,12,12b-octahydroindolo[2,3-a]quinolizine in 1.5 ml. of dry methanol 0.048 g. (0.00126 mole) of sodium borohydride are added in portions, under stirring, in 1.5 hours. After standing overnight, 3 ml. of acetone are added to the solution, which is then stirred for 30 minutes at room temperature, evaporated and a further 3 ml. of acetone are distilled off. The residue is diluted with 3 ml. of water and extracted with three 3-ml. portions of dichloromethane. The dichloromethane solution is dried over solid anhydrous magnesium sulfate, evaporated and the residue is purified by chromatography on 10 g. of silica gel (Geduran Si 60, 0.063-0.200mm.) using cyclohexane containing 10 % of diethylamine as eluant.

Yield: 0.010 g. (83 %) of an oily product solidifying upon standing

$R_f$: 0.49 (Polygram SIL G/$UV_{254}$, cyclohexane containing 10 % of diethylamine, u.v. light).

Claims

1. 12b-substituted 1-hydroxymethyl-octahydroindolo-[2,3-a]-quinolizine derivatives of formula (I)

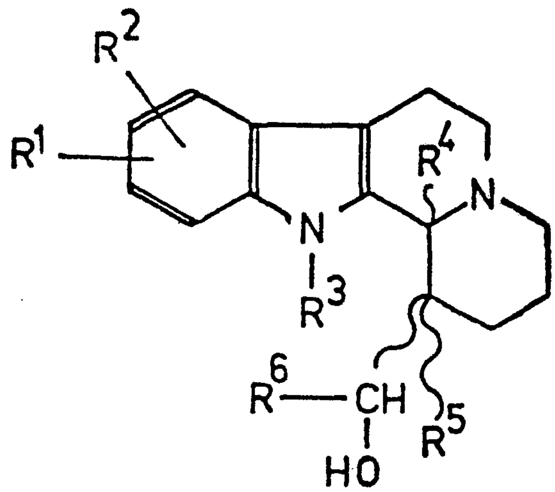

(I)

wherein

$R^1$ and $R^2$ are the same or different and represent hydrogen, halogen, nitro, amino, hydroxyl, alkyl having from 1 to 6 carbon atoms or alkoxy having from 1 to 6 carbon atoms;

$R^3$ and $R^5$ are the same or different and represent hydrogen, alkyl having from 1 to 6 carbon atoms or aralkyl having from 1 to 6 carbon atoms in the alkyl moiety;

$R^4$ is alkyl having from 1 to 6 carbon atoms, aryl or aralkyl having from 1 to 6 carbon atoms in the alkyl moiety;

$R^6$ is hydrogen or alkyl having from 1 to 6 carbon atoms, aryl or aralkyl having from 1 to 6 carbon atoms in the alkyl moiety,

and optically-active isomers, racemic mixtures and acid additional salts thereof.

2. Compounds of formula (I) as claimed in claim 1 selected from the group of:

1-hydroxymethyl-12b-methyl-1,2,3,4,6,7,12,12b-octahydroindolo[2,3-a]quinolizine and its ethanesulfonate;

1-hydroxymethyl-12,12b-dimethyl-1,2,3,4,6,7,12,12b-octahydroindolo[2,3-a]quinolizine;

1-hydroxymethyl-1,12,12b-bimethyl-1,2,3,4,6,7,12,12b-octahydroindolo[2,3-a]quinolizine;

1-(1-hydroxyethyl)-12b-methyl-1,2,3,4,6,7,12,12b-octahydroindolo[2,3-a]quinolizine;

9-bromo-, 10-bromo- and 8,9-dibromo-1-hydroxymethyl-12b-methyl-1,2,3,4,6,7,12,12b-octahydroindolo[2,3-a]quinolizine;

8-nitro- and 10-nitro-1-hydroxymethyl-12b-methyl-1,2,3,4,6,7,12,12b-octahydroindolo[2,3-a]quinolizine; and

10-amino-1-(hydroxymethyl)-12b-methyl-1,2,3,4,6,7,12,-12b-octahydroindolo[2,3-a]quinolizine,

and racemic mixtures and optical antipodes thereof, cis- and trans-isomers thereof, and mixtures of such antipodes and isomers.

3. A pharmaceutical composition comprising as active ingredient at least one optically active 12b-substituted 1-hydroxymethyl-octahydroindolo-[2,3-a]quinolizine derivative of formula (I) or racemic mixtures thereof, as defined in claim 1, or an isomer or a physiologically acceptable acid addition salt thereof, in association with a pharmaceutically acceptable carrier, diluent and/or excipient.

4. A process for the preparation of optically active 12b-substituted 1-hydroxymethyl-octahydroindolo-[2,3-a]quinolizine derivatives as defined in claim 1, comprising

reducing an optically active 1,12b-substituted octahydroindolo-[2,3-a]quinolizine derivative of formula (II)

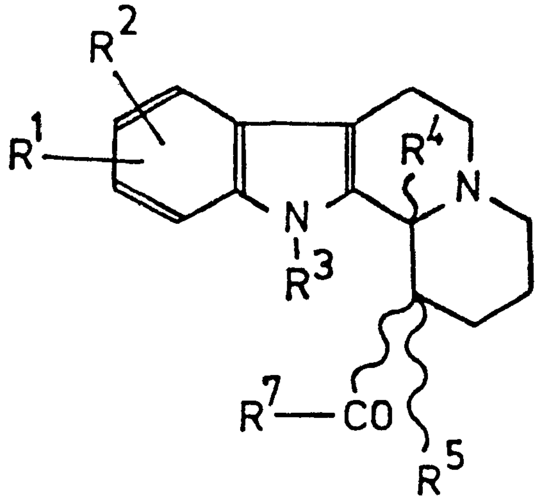

(II)

wherein

$R^1$, $R^2$ are as defined in claim 1 and

$R^7$ is alkoxy having from 1 to 6 carbon atoms, alkyl having from 1 to 6 carbon atoms, aryl or aralkyl having from 1 to 6 carbon atoms in the alkyl moiety,

or racmic mixture thereof; followed, if desired, by

subjecting a 12b-substituted 1-hydroxymethyl-octahydroindolo[2,3-a]quinolizine derivative of formula (I) obtained, in which $R^1$ and/or $R^2$ is halogen or a nitro group and/or $R^3$ represents a benzyl group, and $R^4$, $R^5$ and $R^6$ are as defined in claim 1, to further reduction,

and/or, if desired treating a 12b-substituted 1-hydroxymethyl-octahydroindolo[2,3-a]quinolizine derivative of formula (I) obtained, in which $R^1$, $R^2$, $R^3$, $R^4$, $R^5$ and $R^6$ are as defined in claim 1, with an acid,

or treating an acid addition salt of a 12b-substituted 1-hydroxymethyl-octahydroindolo[2,3-a]quinolizine derivative of formula (I) obtained, wherein $R^1$, $R^2$, $R^3$, $R^4$, $R^5$ and $R^6$ are as defined in claim 1, with a base,

and/or, if desired, separating an isomeric mixture obtained into the respective isomers,

and/or, if desired, resolving a racemic 12b-substituted 1-hydroxymethyl-octahydroindolo[2,3-a]quinolizine derivative of formula (I) obtained.

5. A process as claimed in claim 4, which comprises carrying out the reduction of a 1,12b-disubstituted octahydroindolo[2,3-a]quinolizine derivative of formula (III), wherein

$R^7$ is a alkoxy group having from 1 to 6 carbon atoms,

$R^1$ and $R^2$ are other than halogen or nitro and

$R^3$, $R^4$ and $R^5$ are as defined in claim 1, with lithium aluminium hydride in an inert organic solvent.

6. A process as claimed in either of claims 4 and 5 wherein the reduction is carried out in a linear or cyclic ether or an organic tertiary base.

7. A proces as claimed in claim 4, which comprises carrying out the reduction of a 12b-disubstituted octahydroindolo[2,3-a]quinolizine derivative of formula (II), wherein

$R^7$ is an alkoxy group having from 1 to 6 carbon atoms,

$R^1$ and/or $R^2$ is halogen or nitro and

$R^3$, $R^4$ and $R^5$ are as defined in claim 4, with sodium borohydride, in the present of aluminium chloride, in an inert organic solvent.

8. A process as claimed in claim 4, which comprises carrying out the reduction of a 1,12b-disubstituted

octahydroindolo[2,3-a]quinolizine derivative of formula (II), wherein $R^7$ is alkyl having from 1 to 6 carbon atoms, aryl or aralkyl having from 1 to 6 carbon atoms in the alkyl moiety, $R^1$, $R^2$, $R^3$, $R^4$, and $R^5$ are as defined in claim 4, with sodium borohydride or an aluminium alkoxide having from 1 to 6 carbon atoms, in an inert organic solvent.

9. A process as claimed in claim 4, which comprises carrying out the reduction of a 1,12b-disubstituted octahydroindolo[2,3-a]quinolizine derivative of formula (II), in which $R^7$ is alkyl having from 1 to 6 carbon atoms, aryl or aralkyl having from 1 to 6 carbon atoms in the alkyl moiety, $R^1$ and/or $R^2$ are other than halogen or nitro and/or $R^3$ is other than benzyl, $R^4$ and $R^5$ are as defined in claim 4, in an inert organic solvent by catalytically activated hydrogen.

10. A process as claimed in claim 4 for the preparation of compounds of formula (I), in which $R^1$ and/or $R^2$ is amino, $R^6$ is hydrogen, $R^3$, $R^4$ and $R^5$ are as defined in claim 4, which comprises reducing a compound of formula (II), in which $R^1$ and/or $R^2$ is nitro, $R^7$ is alkoxy, $R^3$, $R^4$ and $R^5$ are as defined in claim 4, with a mixture of lithium aluminium hydride and aluminium chloride.

11. A process as claimed in claim 4 for the preparation of compounds of formula (I), in which $R^1$ and/or $R^2$ is amino, $R^6$ is alkyl having from 1 to 6 carbon atoms in the alkyl moiety, $R^3$, $R^4$ and $R^5$ are as defined in claim 4, which comprises reducing a compound of formula (II), in which $R^1$ and/or $R^2$ is nitro, $R^7$ is identical with $R^6$, $R^3$, $R^4$ and $R^5$ are as defined in claim 4, with catalytically activated hydrogen.

12. A process as claimed in claim 4 for the preparation of compounds of formula (I), in which $R^3$ is hydrogen, $R^1$ and $R^2$ are the same or different and represent hydrogen, amino, hydroxyl, or an alkyl or alkoxy each having from 1 to 6 carbon atoms, $R^6$ is oher than hydrogen, $R^4$, $R^5$ and $R^6$ are as defined in claim 4, which comprises reducing a compound of formula (II), in which $R^3$ in benzyl, $R^1$ and $R^2$ are the same or different and represent hydrogen, halogen, nitro, hydroxyl or alkyl or alkoxy each having from 1 to 6 carbon atoms, $R^4$, $R^5$ and $R^7$ are as defined in claim 4, with catalytically activated hydrogen.

IU 50 450(A)

Claims

1. A process for the preparation of optically active 12b-substituted 1-hydroxymethyl-octahydroindolo-[2,3-a]quinolizine derivatives of formula (I)

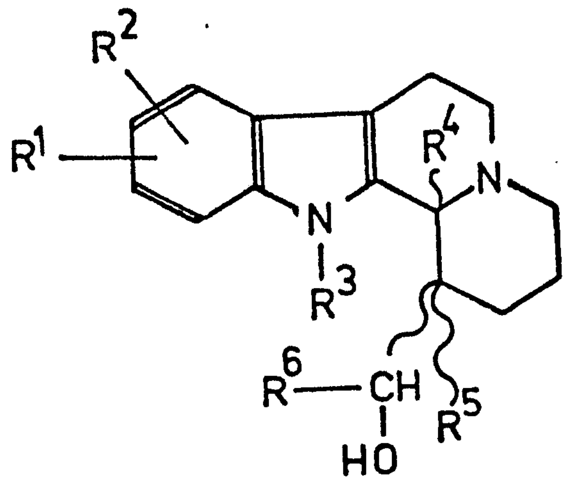

(I)

wherein

$R^1$ and $R^2$ are the same or different and represent hydrogen, halogen, nitro, amino, hydroxyl, alkyl having from 1 to 6 carbon atoms or alkoxy having from 1 to 6 carbon atoms;

$R^3$ and $R^5$ are the same or different and represent hydrogen, alkyl having from 1 to 6 carbon atoms or aralkyl having from 1 to 6 carbon atoms in the alkyl moiety;

$R^4$ is alkyl having from 1 to 6 carbon atoms, aryl or aralkyl having from 1 to 6 carbon atoms in the alkyl moiety;

$R^6$ is hydrogen or alkyl having from 1 to 6 carbon atoms, aryl or aralkyl having from 1 to 6 carbon atoms in the alkyl moiety,

and optically-active isomers, racemic mixtures and acid addition salts thereof comprising

reducing an optically active 1,12b-disubstituted octahydroindolo-[2,3-a]quinolizine derivative of formula (II)

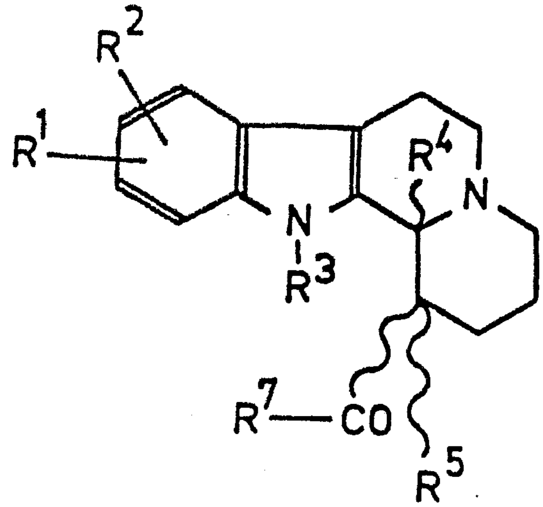

(II)

wherein

$R^1$ and $R^2$ are the same or different and represent hydrogen, halogen, nitro, hydroxyl, alkyl having from 1 to 6 carbon atoms or alkoxy having from 1 to 6 carbon atoms;

and $R^3$, $R^4$, and $R^5$ have the same meanings as defined above,

$R^7$ is a alkoxy group having from 1 to 6 carbon atoms, aryl or aralkyl having from 1 to 6 carbon atoms in the alkyl moiety,

.

or racmic mixture thereof; followed, if desired, by subjecting a 12b-substituted 1-hydroxymethyl-octahydroindolo[2,3-a]quinolizine derivative of formula (I) obtained, in which $R^1$ and/or $R^2$ is halogen or a nitro group and/or $R^3$ represents a benzyl group, and $R^4$, $R^5$ and $R^6$ are as defined in claim 1, to further reduction,

and/or, if desired, treating a 12b-substituted 1-hydroxymethyl-octahydroindolo[2,3-a]quinolizine derivative of formula (I) obtained, in which $R^1$, $R^2$, $R^3$, $R^4$, $R^5$ and $R^6$ are as defined in claim 1, with an acid,

or treating an acid addition salt of a 12b-substituted 1-hydroxymethyl-octahydroindolo[2,3-a]quinolizine derivative of formula (I) obtained, wherein $R^1$, $R^2$, $R^3$, $R^4$, $R^5$ and $R^6$ are as defined in claim 1, with a base,

and/or, if desired, separating an isomeric mixture obtained into the respective isomers,

and/or, if desired, resolving a racemic 12b-substituted 1-hydroxymethyl-octahydroindolo[2,3-a]quinolizine derivative of formula (I) obtained.

2. A process as claimed in claim 1, which comprises carrying out the reduction of a 12b-disubstituted octahydroindolo[2,3-a]quinolizine derivative of formula (II), wherein

$R^7$ is a alkoxy group having from 1 to 6 carbon atoms,

$R^1$ and $R^2$ are other than halogen or nitro, and

$R^3$, $R^4$ and $R^5$ are as defined in claim 1,

with lithium aluminium hydride in an inert organic solvent.

3. A process as claimed in either of claims 1 and 2 wherein the reduction is carried out in a linear or cyclic ether or an organic tertiary base.

4. A process as claimed in claim 1, which comprises carrying out the reduction of a 1,12b-disubstituted octahydroindolo[2,3-a]quinolizine derivative of formula (II), wherein $R^7$ is a alkoxy having from 1 to 6 carbon atoms, $R^1$ and/or $R^2$ is halogen or nitro, $R^3$, $R^4$ and $R^5$ are as defined in claim 1, with sodium borohydride, in the presence of aluminium chloride in an inert organic solvent.

5. A process as claimed in claim 1, which comprises carrying out the reduction of a 1,12b-disubstituted octahydroindolo[2,3-a]quinolizine derivative of formula (II), $R^7$ is alkyl having from 1 to 6 carbon atoms, aryl or aralkyl having from 1 to 6 carbon atoms in the alkyl moiety, $R^1$, $R^2$, $R^3$, $R^4$ and $R^5$ are as defined in claim 1,
with sodium borohydride or an aluminium alkoxide having from 1 to 6 carbon atoms, in an inert organic solvent.

6. A process as claimed in claim 1, which comprises carrying out the reduction of a 12b-disubstituted octahydroindolo[2,3-a]quinolizine derivative of formula (II), in which $R^7$ is alkyl having from 1 to 6 carbon atoms, aryl or aralkyl having from 1 to 6 carbon atoms in the alkyl moiety, $R^1$ and/or $R^2$ are other than halogen or nitro and/or $R^3$ is other than benzyl, $R^4$ and $R^5$ are as defined in claim 1 in an inert organic solvent by catalytically activated hydrogen.

7. A process as claimed in claim 1 for the preparation of compounds of formula (I), in which $R^1$ and/or $R^2$ is amino, $R^6$ is hydrogen, $R^3$, $R^4$ and $R^5$ are as defined in claim 1, which comprises reducing a compound of formula (II), in which $R^1$ and/or $R^2$ is nitro, $R^7$ is alkoxy, $R^3$, $R^4$ and $R^5$ are as defined in claim 1 with a mixture of lithium aluminium hydride and aluminium chloride.

8. A process as claimed in claim 1 for the preparation of compounds of formula (I), in which $R^1$ and/or $R^2$ is amino, $R^6$ is alkyl having having from 1 to 6 carbon atoms, aryl or aralkyl having from 1 to 6 carbon atoms in the alkyl moiety, $R^3$, $R^4$ and $R^5$ are as defined in claim 1, which comprises reducing a compound of formula (II), in which $R^1$ and/or

$R^2$ is nitro, $R^7$ is identical with $R^6$, $R^3$, $R^4$ and $R^5$ are as defined in claim 1 with catalytically activated hydrogen.

9. A process as claimed in claim 1 for the preparation of compounds of formula (I), in which $R^3$, is hydrogen, $R^1$ and $R^2$ are the same or different and represent hydrogen, amino, hydroxyl, or an alkyl or alkoxy each having from 1 to 6 carbon atoms, $R^6$ is oher than hydrogen, $R^4$, $R^5$ and $R^6$ are as defined in claim 1, which comprises reducing a compound of formula (II), in which $R^3$ is benzyl, $R^1$ and $R^2$ are the same or different and represent hydrogen, halogen, nitro, hydroxyl or alkyl or alkoxy each having from 1 to 6 carbon atoms, $R^4$, $R^5$ and $R^7$ are as defined in claim 1, with catalytically activated hydrogen.

10. A process as claimed in any one of claims 8, 10 or 11, in which Raney nickel, palladium-on-charcoal or platinum is employed as a catalyst.

11. A process for the preparation of pharmaceutical compositions, which comprises admixing at least one racmic or optically active 12b-substituted 1-hydroxymethyl-octahydroindolo[2,3-a]quinolizine derivatives of formula (I) as defined in claim 1, isomers or physiologically acceptable acid addition salts thereof, with pharmaceutical carriers and/or excipients.